# EUROPÄISCHE PATENTANMELDUNG

(11) **EP 0 834 500 A1**
(43) Veröffentlichungstag der Anmeldung: **08.04.1998**
(21) Anmeldenummer: 97116483.5
(22) Anmeldetag: 22.09.1997
(51) Int. Cl.: C07C 213/02, C07C 217/04, C07C 217/28

(54) **Verfahren zur Herstellung von Diaminodipropylethern bzw. Hydroxyaminodipropylethern**

(30) Priorität: 04.10.1996 DE 19640975
(71) Anmelder: BAYER AG, 51368 Leverkusen (DE)
(72) Erfinder: Waldmann, Helmut, Dr., 52385 Nideggen (DE); Nachtkamp, Klaus, Dr., 40593 Düsseldorf (DE); Pedain, Josef, Dr., 51061 Köln (DE); Bazanov, Anatoly, Prof, 193318 St. Petersburg (RU); Timofeev, Alexandre, Dr., 195253 St. Petersburg (RU); Zubritskaya, Natalja, Dr., 190068 St. Petersburg (RU); Terechtchenko, Gennady, Prof., 195220 St. Petersburg (RU)

(57) **Zusammenfassung**

Verfahren zur Herstellung Von Diaminodipropylethern bzw. Hydroxyaminodipropylethern durch katalytische Aminierung von Dipropylenglykol.

## Beschreibung

Die Erfindung betrifft ein Verfahren zur Herstellung von Diaminodipropylethern bzw. Hydroxyaminodipropylethern durch katalytische Aminierung von Dipropylenglykol.

Aus der US-PS 36 54 370 ist bekannt, Polyoxyalkylendiamine aus Polyoxyalkylendiolen durch Umsetzung mit Ammoniak und Wasserstoff in Gegenwart von Nickel-Katalysatoren herzustellen. Jedoch wird dabei das Polyoxyalkylen-Gerüst umgelagert (vgl. JP 029 313 (1974)).

Aufgabe dieser Erfindung ist, ein Verfahren bereitzustellen, das Diaminodipropylether und Hydroxyaminodipropylether in guten Ausbeuten liefert, ohne daß die Struktur der Ausgangsverbindungen durch Umlagerung verändert wird.

Gegenstand der Erfindung ist ein Verfahren zur Herstellung Von Diaminodipropylethern bzw. Hydroxyaminodipropylethern, das dadurch gekennzeichnet ist, daß Dipropylenglykol bei einer Temperatur Von 150 bis 190°C und einem Druck von 75 bis 250 bar in Gegenwart eines Ni/Cu/Cr-Katalysators, der 35 bis 58 mol-% Nickel, 10 bis 30 mol-% Kupfer und 12 bis 55 mol-% Chrom enthält, mit Ammoniak und Wasserstoff umgesetzt wird.

Erfindungsgemäß kann beispielsweise handelsübliches Dipropylenglykol (eine Minschung aus den drei Isomeren 1,1'-Oxydi-2-propanol, 2-(2'-Hydroxypropoxy)-1-propanol und 2,2'-Oxydi-1-propanol) zu den entsprechenden Diaminen bzw. Aminoalkoholen umgesetzt werden.

Die Umsetzung kann in einem Durchflußreaktor mit Katalysatorbett ausgeführt werden bei Temperaturen Von 150 bis 190°C und einem Druck von 70 bis 250 bar.

Der verwendete Katalysator ist bevorzugt ein mit Wasserstoff hydriertes Gemisch aus Nickeloxyd, Kupferoxyd und Chromoxyd, welches 35 bis 58 mol-% Nickel, 10 bis 30 mol-% Kupfer und 12 bis 55 mol-% Chrom enthalt. Im allgemeinen wird der Katalysator als Festbett verwendet. Er liegt in Form von Teilchen einer Größe Von ca. 1 bis ca. 10 mm vor, beispielsweise in Form fester Tabletten.

Katalysatoren dieser Art sind prinzipiell bekannt. Sie sind beispielsweise beschrieben in React. Kinet. Catal. Lett., Vol. 44; Nr. 1, 215-222 (1991).

Als Ausgangsmaterial wird reines oder kommerzielles Dipropylenglykol benutzt. Dabei wird durch die Umsetzung mit Wasserstoff und Ammoniak ein Gemisch aus Diaminodipropylether und Hydroxyaminodipropylether im Molverhältnis 1 : 1,5 bis 1 : 20 erhalten. Will man erfindungsgemäß reinen Diaminodipropylether erzeugen, dann kann man als Ausgangsprodukt ein Gemisch aus Dipropylenglykol und Hydroxyaminodipropylether einsetzen.

Die erfindungsgemäß hergestellten Produkte können als Zwischenprodukte zur Herstellung von Kunststoffen verwendet werden.

### Beispiele

### Beispiel 1

Es wurde ein Durchflußreaktor mit fixiertem Katalysatorbett verwendet. In den Reaktor wurden 100 ml eines Katalysators, der 55 mol-% Nickel, 18 mol-% Kupfer und 27 mol-% Chrom enthielt, gegeben. Der Katalysator wurde mit einer Mischung von Stickstoff und Wasserstoff reduziert.

In diesen Reaktor wurden dann pro Stunde 100 l Wasserstoff (gemessen bei Normalbedingungen), 200 ml Ammoniak (flüssig) und 13 ml Dipropylenglykol (flüssig) eingeführt. Die Reaktion wurde bei 170°C unter einem Druck von 200 bar durchgeführt. Das Von Ammoniak befreite Reaktionsprodukt enthielt 14,3 Gew.-% Diaminodipropylether, 22,1 Gew.-% Hydroxyaminodipropylether, 27,4 Gew.-% Dimethylmorpholin, 15,4 Gew.-% Wasser und 20,7 Gew.-% Dipropylenglykol. Der Umsatz von Dipropylenglykol betrug 76,9 % mit einer relativen Selektivität bezüglich Diaminodipropylether Von 21,1 mol-% und einer relativen Selektivität bezüglich Hydcroxyaminodipropylether Von 32,4 mol-%.

Durch rektifizierende Destillation wurde reiner Diaminodipropylether (Siedepunkt 74 bis 75°C bei 13 mbar) und Hydroxyaminodipropylether (Siedepunkt 99 bis 102°C bei 13 mbar) mit mehr als 99 gew.-%iger Reinheit erhalten.

### Beispiel 2

Die Durchführung und der Katalysator entsprachen Beispiel 1. In den Reaktor wurden pro Stunde 100 l Wasserstoff (gemessen bei Normalbedingungen), 200 ml Ammoniak (flüssig) und 13 ml Dipropylenglykol (flüssig) eingeleitet. Die Reaktion wurde bei 150°C unter einem Druck von 200 bar durchgeführt. Die ammoniakfreien Reaktionsprodukte bestanden aus 0,9 Gew.-% Diaminodipropylether, 19,8 Gew.-% Hydroxyaminodipropylether, 3,3 Gew.-% Dimethylmorpholin, 4 Gew.-% Wasser und 72 Gew.-% Dipropylenglykol. Der Umsatz des Dipropylenglykol betrug somit 25,6 Gew.-% mit einer Selektivität bezüglich Diaminodipropylether von 3,1 mol-% und einer relativen Selektivität bezüglich Hydroxyaminodipropylether von 80,5 mol-%.

### Beispiel 3

Das Verfahren wurde wie in Beispiel 1 durchgeführt. In den Reaktor wurden 200 ml Katalysator gegeben, welcher 38 mol-% Nickel, 11 mol-% Kupfer und 51 mol-% Chrom enthielt. Dieser Katalysator wurde, wie in Beispiel 1 mit einer Mischung aus Stickstoff und Wasserstoff reduziert.

In den Reaktor wurden dann pro Stunde 150 l Wasserstoff (gemessen bei Normalbedingungen), 400 ml Ammoniak (flüssig) und 26 ml Dipropylenglykol (flüssig) eingeleitet. Der Reaktion wurde bei 175°C und einem Druck von 200 bar durchgeführt. Die ammoniakfreien Reaktionsprodukte bestanden aus 10,7 Gew.-% Diaminodipropylether, 26,7 Gew.-% Hydroxyaminodipropylether, 26,7 Gew.-% Dimethylmorpholin, 14,9 Gew.-% Wasser und 20,9 Gew.-% von Dipropylenglykol. Der Umsatz von Dipropylenglykol betrug 76,8 Gew.-% mit einer relativen Selektivität bezüglich Diaminodipropylether von 15,7 mol-% und einer relativen Selektivität bezüglich Hydroxyaminodipropylether von 39 mol-%.

### Beispiel 4

Die Durchführung und der Katalysator waren wie in Beispiel 3 mit der Ausnahme, daß anstelle von Dipropylenglykol eine Mischung aus 53 Gew.-% Dipropylenglykol und 47 Gew.-% von aus der Reaktion gewonnenem Hydroxyaminodipropylether eingesetzt wurde. Das ammoniakfreie Reaktionsprodukt enthielt 7,6 Gew.-% Diaminodipropylether, 45,9 Gew.-% Hydroxyaminodipropylether, 10,6 Gew.-% Morpholin, 5,5 Gew.-% Wasser und 30,4 Gew.-% Dipropylenglykol. Der Umsatz von Dipropylenglykol war somit 40;4 Gew.-% mit einer relativen Selektivität bezüglich Diaminodipropylether von 37,7 mol-%.

## Patentansprüche

1. Verfahren zur Herstellung von Diaminodipropylethern bzw. Hydroxyaminodipropylethern aus Dipropylenglykol, dadurch gekennzeichnet, daß bei einer Temperatur von 150 bis 190°C und einem Druck von 75 bis 250 bar in Gegenwart eines Ni/Cu/Cr-Katalysators enthaltend 35 bis 58 mol-% Nickel, 10 bis 30 mol-% Kupfer und 12 bis 55 mol-% Chrom mit Ammoniak und Wasserstoff arbeitet.
